# FASCICULE DE BREVET EUROPEEN

(11) **EP 0 550 366 B1**
(45) Date de publication et mention de la délivrance du brevet: **28.09.1994**
(21) Numéro de dépôt: 92440149.0
(22) Date de dépôt: 29.12.1992
(51) Int. Cl.: F24F 3/16

(54) **Stérilisateur d'air ambiant**
Luftsterilisator
Air steriliser

(30) Priorité: 30.12.1991 US 815936
(43) Date de publication de la demande: 07.07.1993
(73) Titulaire: CLESTRA CLEANROOM, Société Anonyme, F-67200 Strasbourg (FR)
(72) Inventeur: Wetzel, Lawrence E., Cazenovia, NY 13035 (US)
(74) Mandataire: Nuss, Pierre

(56) Documents cités:
- FR-A- 2 187 359
- US-A- 4 210 429

## Description

La présente invention se rapporte à des systèmes pour purifier et stériliser l'air dans un espace fermé selon le préambule de la revendication 1 (voir par example FR-A- 2 187 359, et elle a plus particulièrement trait à un stérilisateur d'air ambiant pouvant être monté sur une paroi d'un local tel que, par exemple, une chambre d'hôpital isolée, un cabinet médical ou un cabinet dentaire. Des éruptions récentes de maladies hautement infectieuses, telles que la tuberculose, se sont traduites par la nécessité de disposer d'appareils pour retenir, par filtration, des particules charriant des micro-organismes générateurs de troubles, tels que des bactéries, des virus ou des spores. Cette nécessité existe, par exemple, dans des lieux dans lesquels la maladie peut aisément se propager d'une personne à l'autre, par exemple une salle d'hôpital pénitentiaire, une chambre isolée d'un hôpital ou d'une résidence, ou bien un local tel qu'un cabinet dentaire pouvant renfermer une grande quantité de particules ou d'aérosols charriés par l'air, qui sont devenus des biocontaminants. Au stade actuel, ce qu'on appelle des "purificateurs" d'air peuvent être du type filtre d'ambiance ou du type électrostatique. Cependant, aucun type ne présente des moyens efficaces pour neutraliser de quelconques contaminants biologiques. Des lampes de stérilisation aux ultraviolets sont quelquefois utilisées dans des installations sanitaires, mais elles doivent être implantées, dans le local, à une certaine distance du patient de telle sorte que leur rayonnement ne vienne pas directement incider sur un patient ou une autre personne. Cela limite leur efficacité, du fait qu'elles se trouvent généralement en dehors du trajet normal de circulation d'air.

Par conséquent, la présente invention a pour objet de fournir un dispositif stérilisateur d'air ambiant qui se prête à une utilisation dans des hôpitaux, des prisons, des cabinets ou appartements médico-professionnels, pour lutter contre des éruptions de maladies en présence de contaminants en suspension dans l'air, charriés par des particules ou des aérosols.

Un autre objet de la présente invention consiste à fournir un tel dispositif, qui soit en mesure d'engendrer une surpression positive ou une dépression négative, en fonction des besoins, pour contrôler une migration de biocontaminants dans un local ou hors de ce dernier.

La présente invention vise, par ailleurs, à fournir un dispositif qui retienne par filtration des particules microscopiques, et qui tue ensuite des bactéries, des spores, des virus, des microbes générateurs de troubles, voire des substances irritantes biologiques telles que du pollen.

Un objet additionnel de la présente invention consiste à fournir un stérilisateur efficace d'air ambiant, qui soit d'une installation et d'un entretien simples.

Ces objets sont atteints par un appareil selon la revendication 1.

De préférence, le groupe de filtration du type HEPA présente un filtre HEPA en quart de cylindre, dont l'axe est agencé horizontalement et se trouve pour l'essentiel au plafond. Cela se traduit par une circulation d'air s'opérant sensiblement vers l'extérieur et vers le bas à partir de l'orifice de sortie du stérilisateur. Un écran protecteur peut être placé sur le côté extérieur du filtre, afin d'obstruer un quelconque trajet direct de la ligne de transmission visible du rayonnement ultraviolet.

Le stérilisateur fonctionne, de préférence, avec un débit d'air d'environ 5,66 à 8,49m³/min (200-300 cfm). Dans un mode de réalisation préférentiel, les lampes de stérilisation aux ultraviolets sont des lampes fluorescentes qui émettent un rayonnement d'environ 254 nm, ce qui est approprié pour tuer tous les micro-organismes sur de petites particules, c'est-à-dire 10 microns ou moins. Les lampes sont implantées de manière à éclairer le côté "sale" des filtres. Le fait que le filtre supérieur du type HEPA soit configuré en un quart de cylindre maximalise l'exposition du côté sale, ou côté afflux, aux UV.

Les objets, caractéristiques et avantages précités de la présente invention, et de nombreux autres encore, seront mis plus amplement en évidence par la description, ci-après, de la forme de réalisation préférentielle conjointement aux dessins annexés.

La figure 1 est une vue en perspective du stérilisateur d'air ambiant selon l'une des formes de réalisation préférentielles de la présente invention.

La figure 2 est une élévation latérale d'un stérilisateur d'air ambiant selon la figure 1.

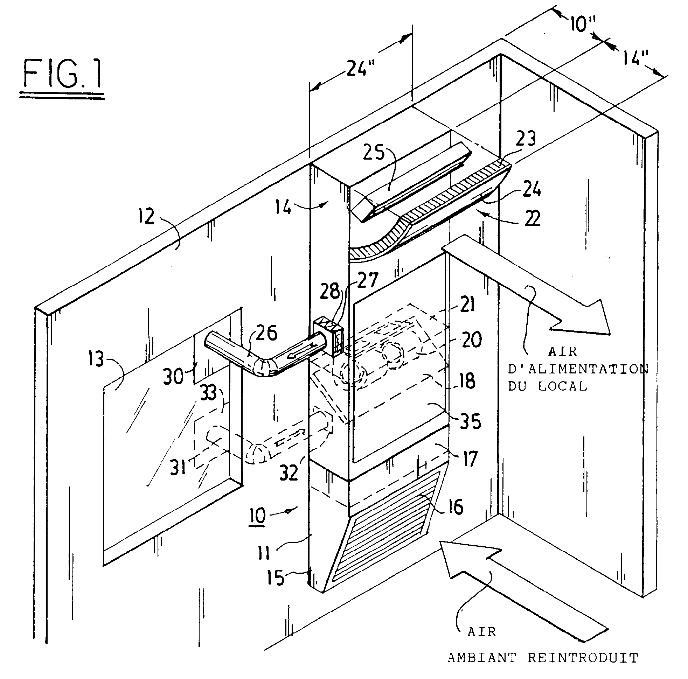

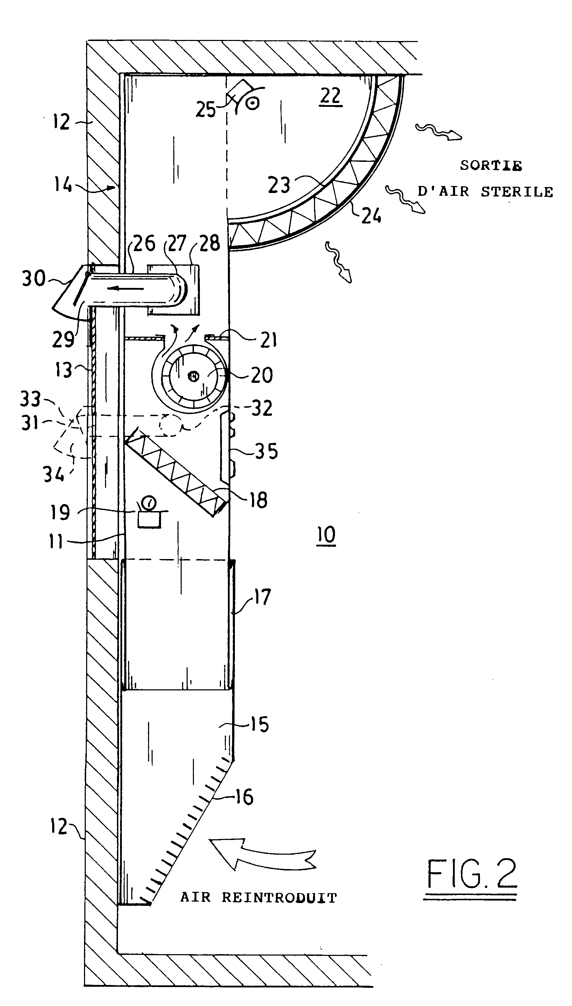

Comme le révèlent les figures 1 et 2 des dessins, auxquelles il convient de se référer, le stérilisateur 10 d'air ambiant conforme à la présente invention comporte un boîtier vertical longiligne 11 de section transversale pour l'essentiel rectangulaire, monté sur une paroi 12 d'un local. Dans ce cas, le stérilisateur 10 est monté à proximité d'une fenêtre 13 ou d'une autre baie pratiquée dans le mur.

Une extrémité supérieure 14 du boîtier est placée contre le plafond du local, tandis qu'une extrémité inférieure 15 se trouve le plus près possible du plancher dudit local. Comme illustré dans ce cas, un intervalle suffisant est réservé, au-dessous de l'extrémité inférieure 15, pour permettre un nettoyage du plancher. Cette forme de réalisation présente une grille 16 à air réintroduit, à travers laquelle de l'air ambiant afflue dans l'extrémité inférieure du boîtier. Toutefois, dans d'autres formes de réalisation possibles, le boîtier pourrait être directement raccordé à un collecteur, à l'intérieur d'un faux-plancher du local.

Un coffrage 17, pouvant être prolongé, permet d'ajuster l'assemblage complet du boîtier 11 de façon qu'il corresponde à la hauteur sous plafond du local.

Un filtre préalable 18, éliminant des particules de poussière grossières, est logé dans le boîtier au-dessus de la grille 16 à air réintroduit. Une lampe 19 de stérilisation aux ultraviolets, située au-dessous du filtre préalable, expose la surface inférieure ou surface d'admission de ce filtre préalable 18 à une lumière ultraviolette d'environ 254 nm. Cela a pour effet de détruire une quelconque contamination biologique sur les particules piégées sur le filtre préalable, et cela permet audit filtre préalable d'être chargé sans qu'il soit nécessaire de prévoir un chemisage, ou de prendre d'autres mesures inhabituelles.

Un ou plusieurs ventilateurs 20 sont disposés dans un carter 21 ou un diaphragme en acier, au-dessus du filtre préalable 18. Ces ventilateurs servent à attirer l'air ambiant réintroduit à travers la grille 16, et à provoquer une circulation forcée de cet air, vers le haut, jusqu'à un orifice de sortie 22 à l'extrémité supérieure 14 du boîtier. Dans cette forme de réalisation, le stérilisateur d'air ambiant utilise une paire de ventilateurs centrifuges silencieux, logés dans des compartiments spiroïdaux ou hélicoïdaux. Ces ventilateurs présentent, associativement, une capacité de brassage d'air d'environ 8.49 m³/min (300 cfm).

Un filtre 23 du type HEPA, par l'intermédiaire duquel de l'air stérile d'alimentation du local est expulsé dans ce local, se trouve dans la région de l'orifice de sortie 22. Le filtre 23 revêt, dans ce cas, la forme d'un quart de cylindre dont l'axe (c'est-à-dire le centre de courbure) est agencé horizontalement et pour l'essentiel à la hauteur du plafond, de telle sorte que l'air, expulsé à travers le filtre, circule vers l'extérieur et vers le bas jusque dans le local. Un écran protecteur 24, se présentant comme une double rangée de lattes, est installé à l'extérieur du filtre 23.

Un lampe 25 de stérilisation aux ultraviolets est logée à l'intérieur de la partie supérieure 14 du boîtier, et est conçue pour exposer toute la surface interne, c'est-à-dire le côté afflux du filtre 23, à une lumière ultraviolette germicide. Cette lumière présente, de préférence, une longueur d'ondes d'environ 254 nm. Le filtre 23 du type HEPA emprisonne des particules en suspension dans l'air et d'autres particules dont la taille atteint jusqu'à un micron ou moins, et la lampe de stérilisation 25 détruit une quelconque contamination biologique sur les particules piégées sur le filtre. L'écran protecteur 24 à lattes doubles autorise une libre sortie de l'air à travers le filtre 23, mais obstrue la ligne de transmission visible d'un quelconque rayonnement ultraviolet susceptible de traverser le filtre.

La pression relative de l'air ambiant peut être commandée , au moyen de clapets et de conduits raccordés au boîtier 11, afin d'engendrer soit une légère surpression, soit une légère dépression par rapport à l'espace extérieur au local.

Pour une pression relative négative, un conduit d'expulsion 26 est raccordé, par l'une de ses extrémités, à un orifice d'expulsion qui est pratiqué, dans le boîtier, entre le carter 21 des ventilateurs et le filtre 23. L'autre extrémité du conduit 26 gagne la fenêtre 13 ou autre baie ménagée dans le mur. Un filtre facultatif 28 du type HEPA peut être implanté dans la région de l'orifice d'expulsion 27, et a pour objet d'empêcher une contamination de l'air extérieur. Comme illustré sur la figure 2, un clapet anti-retour 29 d'équilibrage de l'expulsion est situé à l'extrémité expulsion du conduit 26, à laquelle une coiffe protectrice 30 est également prévue. Le conduit d'expulsion 26 peut également renfermer des clapets manuels, non représentés.

Comme illustré par des pointillés, un conduit d'admission 31 peut être prévu pour obtenir une pressurisation positive ou une surpression du local. Dans ce cas, le conduit d'admission 31 est raccordé à un orifice d'admission 32 pratiqué en avant des ventilateurs 20, c'est-à-dire du côté admission de ces derniers. Comme le montre la figure 2, le conduit 31 s'étend jusqu'à une coiffe protectrice 33 située dans la fenêtre 13, et dans laquelle un clapet antiretour d'admission 34 est intégré pour commander la circulation d'air parcourant ledit conduit. Les flèches, tracées sur les conduits 26 et 31, indiquent la direction de la circulation d'air les parcourant.

Dans cette forme de réalisation, un panneau de commande 35 se trouve à l'avant du boîtier 11. Le panneau de commande comprend des interrupteurs destinés aux ventilateurs et aux lampes, ainsi que des témoins conçus pour indiquer l'état de fonctionnement des lampes 19 et 25, et également pour indiquer, par exemple, une chute de pression se produisant en travers des filtres 18 et 23. Des témoins supplémentaires peuvent être utilisés pour indiquer le débit d'air, le comptage des particules, la température, l'humidité et/ou autres grandeurs. Le stérilisateur 10 d'air ambiant selon cette forme de réalisation est un dispositif réclamant une faible puissance, et peut être enfiché dans n'importe quelle prise normalisée de la paroi.

Dans des variantes de réalisation, au lieu de raccorder les conduits d'expulsion 26 et d'admission 31 à travers une fenêtre ou autre baie pratiquée dans le mur, ces conduits peuvent être raccordés à un canal collecteur commun du système de ventilation de l'immeuble. De plus, au lieu de s'achever au-dessus du sol, l'extrémité inférieure du boîtier peut gagner un collecteur à l'intérieur d'un faux-plancher, du local. Dans ce cas, des perforations ménagées dans le faux-plancher peuvent autoriser, dans le local, une circulation d'air sensiblement laminaire qui peut favoriser un piégeage de particules en suspension dans l'air.

## Revendications

1. Stérilisateur d'air ambiant pour retenir, par filtration, des particules et des aérosols renfermés par l'air d'un local, et pour détruire une biocontamination microbienne sur ces derniers, dans lequel un boîtier longiligne (11) comprend une extrémité(15) d'air réintroduit, percée d'un orifice (16) d'air réintroduit pour attirer de l'air réintroduit à partir d'un emplacement proche du sol dudit local, et une extrémité de décharge (14) percée d'un orifice de sortie (22) ; un ventilateur (20), situé dans ledit boîtier, provoque une circulation forcée de l'air, dudit orifice d'air réintroduit jusqu'audit orifice de sortie ; un groupe de filtration (23) du type HEPA, installé dans la région dudit orifice de sortie, piège et retient lesdites particules et lesdits aérosols lorsque ledit air circule, à travers ce groupe, d'un côté afflux vers un côté sortie de ce dernier ; et une lampe germicide (25) à ultraviolets se trouve dans ledit boîtier, à son extrémité de décharge (14), afin d'exposer le côté afflux dudit groupe de filtration (23) du type HEPA, dans son intrégalité, à un rayonnement ultraviolet d'une longueur d'ondes adéquate pour détruire ladite biocontamination microbienne ; caractérisé par le fait qu'un filtre préalable (18) est logé dans ledit boîtier, en amont dudit ventilateur (20), de telle sorte qu'une zone de pression réduite soit définie entre le filtre préalable et le ventilateur (20), et qu'une zone de pression accrue soit définie entre le ventilateur et ledit filtre (23) du type HEPA ; un orifice d'admission (32) ménagé dans le boîtier communique avec ladite zone de pression réduite, et un orifice d'expulsion (28) pratiqué dans le boîtier communique avec ladite zone de pression accrue ; et un conduit (26) est sélectivement raccordé soit à l'orifice d'admission, soit à l'orifice d'expulsion, pour introduire de l'air conditionné dans le local ou pour expulser de l'air hors dudit local, et peut fonctionner sélectivement en un mode surpression dans lequel de l'air conditionné circule par ledit conduit (26) et par ledit orifice d'admission (32), afin d'entretenir une pression positive dans ledit local, et en un mode dépression dans lequel de l'air de sortie est évacué à l'extérieur du local, par l'intermédiaire dudit orifice d'expulsion (27) et dudit conduit (26), afin d'entretenir une pression négative à l'intérieur du local.

2. Stérilisateur d'air ambiant, selon la revendication 1, caractérisé par le fait que ledit boîtier présente un prolongement de conduit (17) de hauteur variable, entre ledit orifice (16) d'air réintroduit et ledit ventilateur (20), pour permettre audit orifice d'air réintroduit d'être placé au voisinage direct du sol.

3. Stérilisateur d'air ambiant, selon la revendication 1, caractérisé, en outre, par le fait qu'une seconde lampe germicide (19) à ultraviolets est située au-dessous dudit filtre préalable (18), afin d'exposer un côté afflux de ce dernier à un rayonnement ultraviolet présentant ladite longueur d'ondes adéquate.

4. Stérilisateur d'air ambiant, selon la revendication 1, caractérisé, en outre, par le fait que ledit groupe de filtration (23) du type HEPA présente un filtre en quart de cylindre dont l'axe est agencé horizontalement, pour l'essentiel, au plafond précité.

5. Stérilisateur d'air ambiant, selon la revendication 1, caractérisé, en outre, par le fait qu'un écran frontal protecteur, formé par des rangées étagées de lattes (24), est installé sur le côté sortie dudit filtre (23) du type HEPA.

6. Stérilisateur d'air ambiant, selon la revendication 1, caractérisé, en outre, par le fait que le conduit (26) renferme un filtre d'expulsion (28) situé dans la région dudit orifice d'expulsion.

7. Stérilisateur d'air ambiant, selon la revendication 1, caractérisé, en outre, par le fait que ledit conduit (26) renferme un clapet antiretour (29) pour bloquer la circulation d'air qui le parcourt.

## Patentansprüche

1. Umgebungsluftsterilisator zum Zurückhalten von Partikeln und Aerosolen welche in der Luft eines Raumes enthalten sind durch Filtration, und zum Zerstören einer biologischen Kontamination durch Mikroben aufletzteren, wobei im Sterilisator ein längliches Gehäuse (11) ein Ende (15) für zufließende Luft besitzt, versehen mit Lufteintritts-Öffnungen (16), um eintretende Luft von einer bodennahen Stelle des genannten Raumes aus anzusaugen, und ein Luftaustritts-Ende (14), versehen mit einer Austrittsöffnung (22);
ein im genannten Gehäuse befindlicher Ventilator (20) bewirkt eine verstärkte Luftzirkulation von der genannten Lufteintritts-Öffnung bis zur genannten Luftaustritts-Öffnung;
eine Filtereinheit (23) vom Typ HEPA (=filtre a haute efficacite pour particules en suspension dans l' air = Filter mit hoher Leistung für in Luft schwebende Partikel), welche in der Nähe der genannten Austrittsöffnung angeordnet ist, fängt die genannten Partikel und die genannten Aerosole ein und hält sie zurück, da die genannte Luft durch diese Einheit (23) hindurch von der Zustrom-Seite zu einer Austritts-Seite hieraus stömt; und eine keimtötende Ultraviolett-Lampe befindet sich am Austrittsende (14) im genannten Gehäuse, damit die Zustromseite der genannten Filtereinheit (23) vom Typ HEPA in ihrer Gesamtheit einer ultravioletten Strahlung ausgesetzt wird, welche die geeignete Wellenlänge zur Zerstörung der biologischen Kontamination durch Mikroben aufweist;
dadurch gekennzeichnet, daß ein Vorfilter (18) sich in genanntem Gehäuse oberhalb des genannten Ventilators (20) befindet, und zwar derart, daß eine Zone verminderten Drucks zwischen dem Vorfilter und dem Ventilator (20) entsteht, und daß eine Zone erhöhten Drucks zwischen dem Ventilator und dem genannten Filter (23) vom Typ HEPA entsteht; eine im Gehäuse angebrachte Zufuhr- Öffnung (32) welche mit der genannten Zone reduzierten Drucks verbunden ist, und eine Austritts- Öffnung (28) im Gehäuse, welche mit der genannten Zone erhöhten Drucks verbunden ist; und eine Röhre (26) ist wahlweise entweder mit der Zufuhr-Öffnung oder mit der Austritts-Öffnung verbunden, um die konditionierte Luft in den Raum einzubringen oder die Luft aus dem genannten Raum auszustoßen, und kann wahlweise entweder in einem Überdruck-Modus arbeiten, in welchem konditionierte Luft durch die genannte Röhre (26) und durch die genannte Zuführ-Öffnung (32) stömt, um einen positiven Druck im genannten Raum aufrechtzuerhalten, oder in einem Unterdruck-Modus in welchem Luft aus dem Raum nach draußen evakuiert wird, und zwar durch genannte Austritts-Öffnung (27) und durch genannte Röhre (26), um einen negativen Druck im Inneren des Raumes aufrechtzuerhalten.

2. Umgebungsluftsterilisator nach Anspruch 1, dadurch gekennzeichnet, daß das genannte Gehäuse eine röhrenförmige (17) Verlängerung variabler Höhe zwischen der genannten Lufteintritts-Öffnung (16) und dem genannten Ventilator (20) aufweist,um das Anbringen der genannten Lufteintritts-Öffnung direkt in der Nähe des Fußboden zu ermöglichen.

3. Umgebungsluftsterilisator nach Anspruch 1, dadurch gekennzeichnet, daß überdies eine zweite keimtötende Ultraviolett-Larnpe (19) unterhalb des genannten Vorfilters (18) angebracht ist, um eine Zustrom-Seite des letzteren einer ultravioletten Bestrahlung mit genannter Wellenlänge auszusetzen.

4. Umgebungsluftsterilisator nach Anspruch 1, dadurch gekennzeichnet, daß überdies dadurch, daß die genannte Filtereinheit (23) des Typs HEPA einen Filter in der grundlegenden Form eines Viertel- Zylinders aufweist, dessen Achse waagrecht an der obenerwähnten Decke angeordnet ist.

5. Umgebungsluftsterilisator nach Anspruch 1, dadurch gekennzeichnet, daß überdies eine vorderseitige schützende Abschirmung, welche ein Lattengestell (24) gebildet wird, auf der Austrittsseite des genannten Filters (23) des Typs HEPA angebracht ist.

6. Umgebungsluftsterilisator nach Anspruch 1, dadurch gekennzeichnet, daß überdies die Röhre (26) einen Austritts- Filter (28) umfaßt, der sich in der Nähe der genannten Austritts-Öffnung (28) befindet.

7. Umgebungsluftsterilisator nach Anspruch 1, dadurch gekennzeichnet, daß überdies die genannte Röhre (26) eine Anti- Rückschlag- Klappe (29) aufweist, um das Zurückströmen von durchströmender Luft zu verhindern.

## Claims

1. Ambient air sterilizer for retaining, by filtration, particles and aerosols contained by the air in a room and for destroying microbic biocontamination thereon, in which an elongate casing (11) comprises one end (15) for reintroduced air, which is perforated by an orifice (16) for reintroduced air to attract the reintroduced air from a location close to the floor of said room, and a discharge end (14) perforated by an outlet orifice (22); a fan (20) located in said casing causes forced air circulation from said reintroduced air orifice to said outlet orifice; a filtration unit (23) of the HEPA type installed in the region of said outlet orifice traps and retains said particles and said aerosols when said air circulates through this unit from an influx side to an outlet side thereof; and an ultraviolet germicidal lamp (25) is located in said casing at its discharge end (14) so as to expose the influx side of said filtration unit (23) of the HEPA type in its entirety to ultraviolet radiation having an adequate wavelength to destroy said microbic biocontamination; characterised in that a preliminary filter (18) is accommodated in said casing upstream of said fan (20) so as to define a reduced pressure region between the preliminary filter and the fan (20) and an increased pressure region is defined between the fan and said filter (23) of the HEPA type; an intake orifice (32) made in the casing communicates with said reduced pressure region and a delivery orifice (28) made in the casing communicates with said increased pressure region; and a conduit (26) is selectively connected either to the intake orifice or to the delivery orifice so as to introduce conditioned air into the room or to expel air out of said room and can operate selectively in a superpressure mode in which the conditioned air circulates through said conduit (26) and through said intake orifice (32) so as to maintain a positive pressure in said room and in a depression mode in which the issuing air is evacuated to the exterior of the room via said delivery orifice (27) and said conduit (26) so as to maintain a negative pressure inside the room.

2. Ambient air sterilizer according to claim 1, characterised in that said casing has a conduit extension (17) of variable height between said reintroduced air orifice (16) and said fan (20) to allow said reintroduced air orifice to be placed in the immediate vicinity of the floor.

3. Ambient air sterilizer according to claim 1, characterised, furthermore, in that a second ultraviolet germicidal lamp (19) is located below said preliminary filter (18) so as to expose an influx side thereof to ultraviolet radiation of said adequate wavelength.

4. Ambient air sterilizer according to claim 1, characterised, furthermore, in that said filtration unit (23) of the HEPA type has a filter in the form of a quarter cylinder of which the axis is arranged substantially horizontally to the aforementioned ceiling.

5. Ambient air sterilizer according to claim 1, characterised, furthermore, in that a protective front screen formed by layered rows of battens (24) is installed on the outlet side of said filter (23) of the HEPA type.

6. Ambient air sterilizer according to claim 1, characterised, furthermore, in that the conduit (26) contains a delivery filter (28) located in the region of said delivery orifice.

7. Ambient air sterilizer according to claim 1, characterised, furthermore, in that said conduit (26) contains a nonreturn valve (29) to block the circulation of air traversing it.
